# EUROPEAN PATENT SPECIFICATION

(11) **EP 2 476 361 B1**
(45) Date of publication and mention of the grant of the patent: **25.09.2013**
(21) Application number: 11194479.9
(22) Date of filing: 20.12.2011
(51) Int. Cl.: A61B 1/00

(54) **Guide assembly for endoscope**
Führungsanordnung für Endoskop
Assemblage guide pour endoscope

(30) Priority: 13.01.2011 JP 2011004995
(43) Date of publication of application: 18.07.2012
(73) Proprietor: Fujifilm Corporation, Minato-ku Tokyo (JP)
(72) Inventor: Yamakawa, Shinichi, Kanagawa (JP); Ashida, Tsuyoshi, Kanagawa (JP); Nakamura, Takayuki, Kanagawa (JP); Ohta, Yasunori, Kanagawa (JP); Iwasaka, Masayuki, Kanagawa (JP)
(74) Representative: Klunker . Schmitt-Nilson . Hirsch

(56) References cited:
- US-A1- 2002 173 700
- US-A1- 2004 204 702
- US-A1- 2005 154 278
- US-A1- 2005 272 976

## Description

### BACKGROUND OF THE INVENTION

### 1. Field of the Invention

The present invention relates to a guide assembly for an endoscope. More particularly, the present invention relates to a guide assembly for propelling an elongated tube of an endoscope into a body cavity, and in which a cavity wall of a body cavity accessed by the endoscope can be protected even in propulsion of the guide assembly.

### 2. Description Related to the Prior Art

A diagnosis by use of an endoscope is well-known in the field of the medicine. The endoscope includes a head assembly with a CCD or other image sensor, and a section of an elongated tube where the head assembly is disposed at its distal end. The elongated tube is entered in a body of a patient. An image is obtained by the image sensor. A display panel is driven to display the image, for imaging of an object in the body.

A guide assembly for use with the endoscope by assisting the entry in a body cavity has been suggested recently. U. S. Pat. Ser.No. 2005/0272976 (corresponding to JP-A 2005-253892) discloses the guide assembly including a sleeve for mounting on the elongated tube of the endoscope, and endless belts secured to the sleeve in a circulating manner. An outer surface of the endless belts is set in contact with a cavity wall of a body cavity or gastrointestinal tract, before the endless belts are turned around to propel the endoscope into the body cavity by use of friction with a cavity wall of the body cavity. This is effective in facilitating entry of the endoscope into the body cavity such as a large intestine which is an organ of a highly tortuous shape, specifically for an unskilled medical service provider in relation of manipulating the endoscope.

In U.S. Pat. Ser.No. 2005/0272976, an upper belt run of the endless belts is nearly flat. A pair of lateral side portions of the endless belts appear externally. As a shape of the body cavity or gastrointestinal tract is nearly circular as viewed in a cross section, the lateral side portions of the endless belts are likely to contact the cavity wall of the body cavity without a contact of its center portion in a transverse direction with the cavity wall. There is a problem of occurrence of entanglement of the cavity wall of the body cavity in an area of an inner surface of the endless belts for damaging upon the contact of the lateral side portions with the cavity wall of the body cavity.

### SUMMARY OF THE INVENTION

In view of the foregoing problems, an object of the present invention is to provide a guide assembly for propelling an elongated tube of an endoscope into a body cavity, and in which a cavity wall of a body cavity accessed by the endoscope can be protected even in propulsion of the guide assembly.

In order to achieve the above and other objects and advantages of this invention, a guide assembly for an endoscope including a section of an elongated tube for entry in a body cavity is provided. There is a shaft sleeve for mounting on the elongated tube. A housing sleeve is supported around the shaft sleeve. Plural endless belts are secured to the housing sleeve, for endlessly moving in an axial direction of the elongated tube, and propelling the elongated tube by contacting a cavity wall of the body cavity. A driving device is contained in the housing sleeve, for driving the endless belts. First and second side rail portions are disposed on the housing sleeve, for covering lateral side portions of each of the endless belts at least partially, and guiding the endless belts.

The first and second side rail portions cause curving of a center portion of the endless belts outwards in a raised form with respect to a transverse direction thereof.

Furthermore, a centrally raised support wall is formed together with the first and second side rail portions, for raising a center portion of the endless belts with respect to a transverse direction thereof outwards further than the lateral side portions.

The side rail portions are formed together with the housing sleeve.

In one preferred embodiment, the side rail portions are secured to the housing sleeve.

Furthermore, plural belt rollers are secured to the housing sleeve in a rotatable manner, for contacting each of the endless belts in a circulating manner in the axial direction.

The support wall projects outwards further than the first and second side rail portions.

Each of the first and second side rail portions includes a side projection disposed to project from the housing sleeve outwards and in a transverse direction of the endless belts, for regulating an outer surface of the lateral side portions.

In another preferred embodiment, each of the first and second side rail portions includes a side rail channel, formed in the housing sleeve, for receiving entry of one of the lateral side portions.

The side rail channel has first and second inner wall surfaces, the first inner wall surface is opposed to an inner surface of the lateral side portions, the second inner wall surface contacts an outer surface of the lateral side portions, and is inclined gradually deeply according to a distance from a center portion of the endless belts.

Each of the endless belts includes an upper belt run, having an outer surface, for contacting the cavity wall. A lower belt run is disposed inside and along the upper belt run, and nipped between the driving device and the belt rollers.

A portion of the endless belts in contact with the belt rollers has higher rigidity than a remaining portion thereof.

The driving device includes a drive sleeve, contained in the housing sleeve, and supported around the shaft sleeve in a rotatable manner. Worm gear teeth are formed around the drive sleeve. An engagement roller has gear teeth, meshed with the worm gear teeth, for turning around the endless belts by engagement therewith.

A shape of the engagement roller is curved according to a curve of the endless belts.

A shape of the belt rollers is curved according to a curve of the endless belts.

The housing sleeve is substantially cylindrical.

In one preferred embodiment, the housing sleeve includes substantially flat N side walls, where N is an integer. N arcuately curved walls are disposed alternately with the N side walls, for supporting respectively the endless belts.

In another preferred embodiment, the housing sleeve includes N arcuately curved walls, where N is an integer. N side walls are disposed alternately with the N curved walls, have a substantially flat inner surface, for supporting respectively the endless belts.

Accordingly, a cavity wall of a body cavity accessed by the endoscope can be protected from interference of endless belts even in propulsion of the guide assembly, because the side rail portions can operate for regulating the lateral side portions of the endless belts.

### BRIEF DESCRIPTION OF THE DRAWINGS

The above objects and advantages of the present invention will become more apparent from the following detailed description when read in connection with the accompanying drawings, in which:
Fig. 1 is a plan illustrating an endoscope;
Fig. 2 is a perspective view illustrating a guide assembly;
Fig. 3 is an exploded perspective view illustrating the guide assembly;
Fig. 4 is a vertical section illustrating the guide assembly;
Fig. 5 is a cross section illustrating components of the guide assembly including belt rollers;
Fig. 6 is a cross section illustrating components of the guide assembly including engagement rollers;
Fig. 7 is a perspective view illustrating one preferred guide assembly of which a housing sleeve is cylindrical;
Fig. 8 is an exploded perspective view illustrating the guide assembly;
Fig. 9 is a cross section illustrating components of the guide assembly including belt rollers;
Fig. 10 is a cross section illustrating components of the guide assembly including engagement rollers;
Fig. 11 is a perspective view illustrating another preferred guide assembly
Fig. 12 is an exploded perspective view illustrating of which a housing sleeve is prismatic;
Fig. 13 is a cross section illustrating components of the guide assembly including belt rollers;
Fig. 14 is a cross section illustrating components of the guide assembly including engagement rollers;
Fig. 15 is a cross section illustrating still another preferred guide assembly with side rail channels.

### DETAILED DESCRIPTION OF THE PREFERRED EMBODIMENT(S) OF THE PRESENT INVENTION

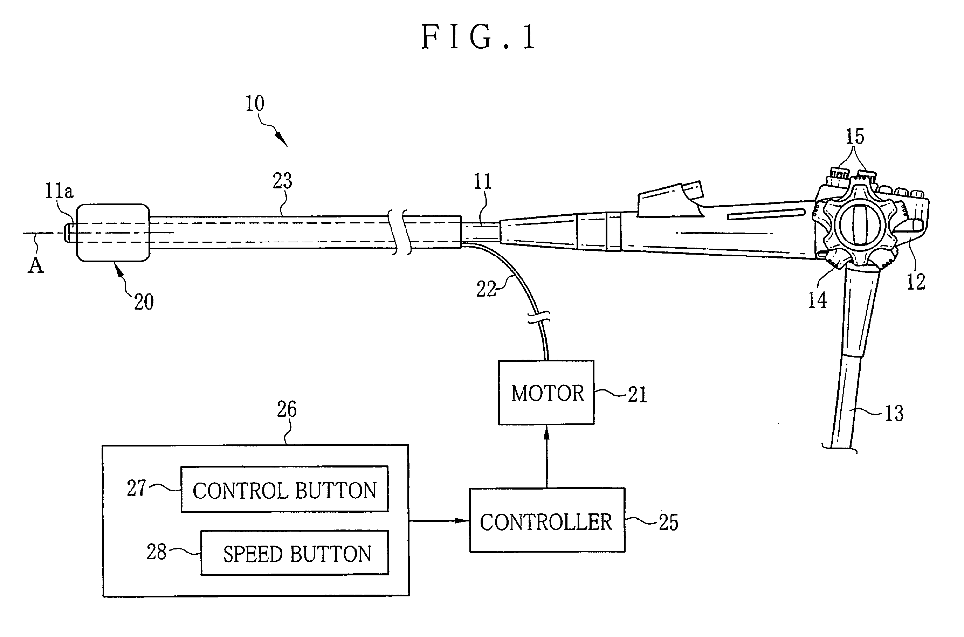

In Fig. 1, an endoscope 10 includes a section of an elongated tube 11 or guide tube, a handle device 12 and a universal cable 13. The elongated tube 11 has the image pickup device in a head assembly 11a, such as a CCD sensor or CMOS sensor of a micro size. The handle device 12 is used for grasping the endoscope 10 and operating the elongated tube 11. The universal cable 13 connects the endoscope 10 to a processing apparatus, a light source apparatus (not shown) and the like.

The elongated tube 11 is a flexible tube of a great length. The head assembly 11a is disposed at a distal end of the elongated tube 11. Various openings are formed in an end surface of the head assembly 11a, including an imaging window, lighting window, end nozzle (all not shown) and the like. The handle device 12 includes steering wheels 14 and flow control buttons 15. The steering wheels 14 are rotated to change a direction and an amount of steering. The flow control buttons 15 are used for supply of air or water and for suction.

The universal cable 13 is connected to the handle device 12. The universal cable 13 includes a signal cable, a light guide device, and a flow channel. The signal cable transmits an image signal from the image pickup device. The flow channel is formed to supply air or water to the head assembly 11a.

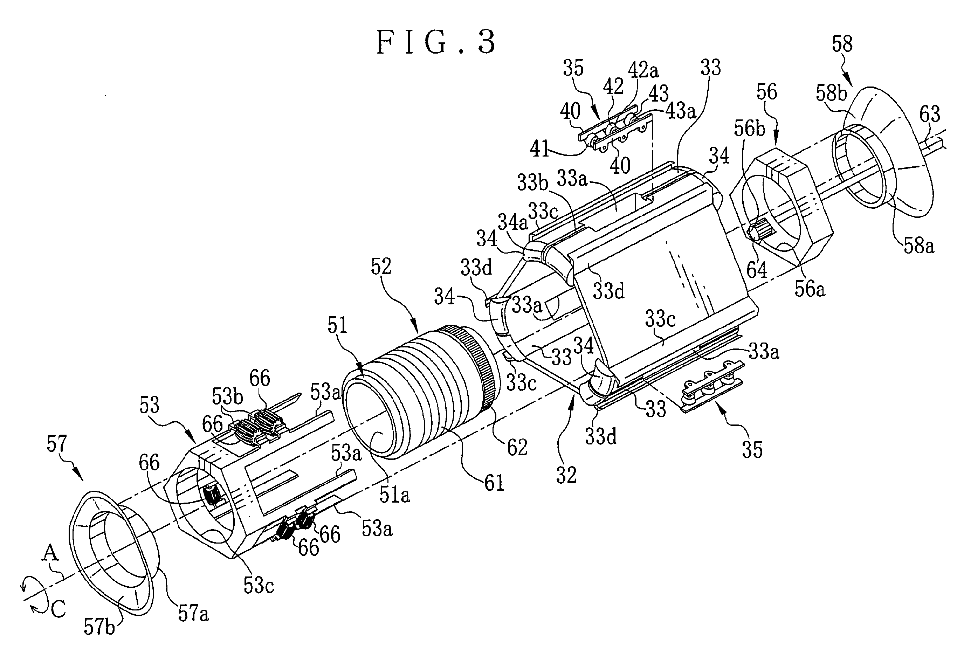

A guide assembly 20 is mounted on the head assembly 11a of the elongated tube 11 in a removable manner, and causes the elongated tube 11 to move in proximal and distal directions in the body cavity. A motor 21 drives the guide assembly 20. A coil structure 63 or torque wire is connected to an output shaft of the motor 21, and transmits torque to rotate for propulsion of the guide assembly 20. See Fig. 3. A protection sheath 22 covers the whole of the coil structure 63. The coil structure 63 rotates in the protection sheath 22 when the motor 21 rotates.

A controller 25 controls the motor 21. An input panel 26 or user interface is connected to the controller 25. The input panel 26 includes a control button 27 and a speed button 28. The control button 27 is operable for inputting command signals for advance, return and stop of the guide assembly 20. The speed button 28 is operable for changing a speed of propulsion of the guide assembly 20.

An overtube 23 is used, in which the elongated tube 11 is mounted. The protection sheath 22 is disposed between the overtube 23 and the elongated tube 11 to extend through.

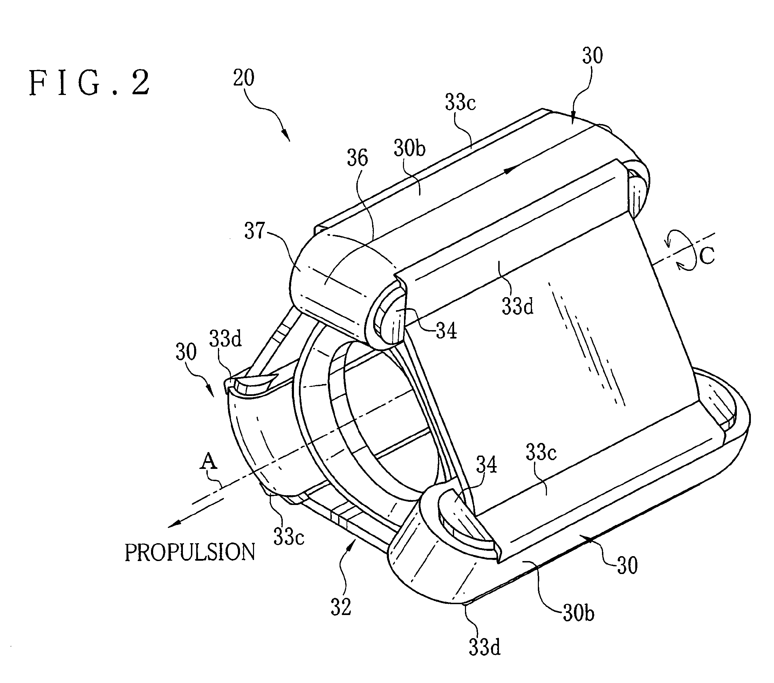

In Fig. 2, plural endless belts 30 are disposed in the guide assembly 20, contact a cavity wall of the gastrointestinal tract, and apply force to the cavity wall in a proximal direction which is reverse to the distal direction of the elongated tube 11 according to an axial direction A of the endoscope 10. A housing sleeve 32 supports the endless belts 30 to circulate in the axial direction A. The endless belts 30 are arranged rotationally equidistantly in a rotational direction C defined about the axis of the axial direction A. An example of the number of the endless belts 30 is three. An outer surface 30b of an upper belt run of the endless belts 30 contacts a cavity wall of the gastrointestinal tract. The arrow in Fig. 2 indicates a direction of the circulation. A material for forming the endless belts 30 is biocompatible plastic material having flexibility, such as polyvinyl chloride (PVC), polyamide resin, fluorocarbon resin, polyurethane and the like.

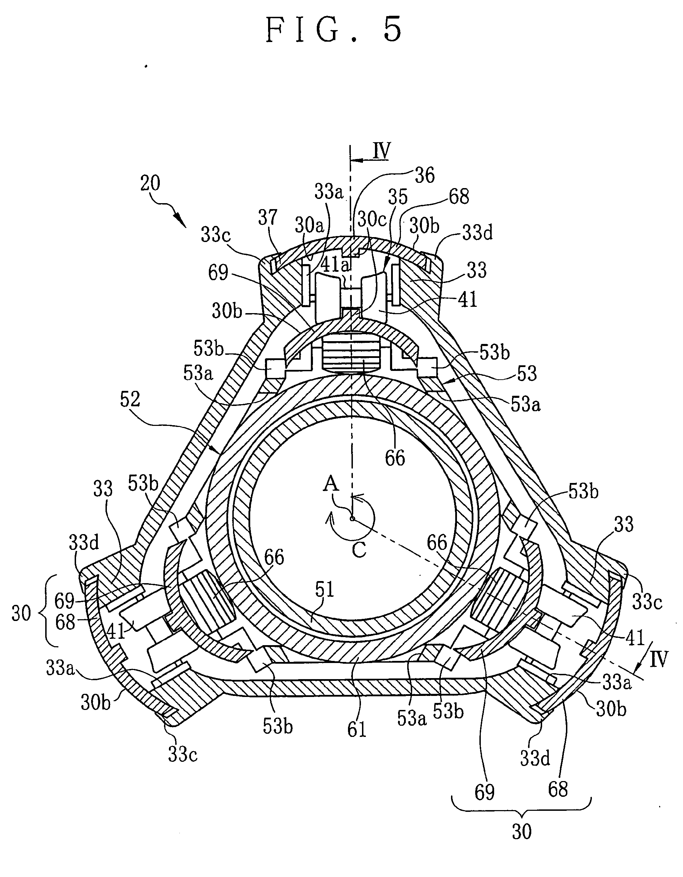

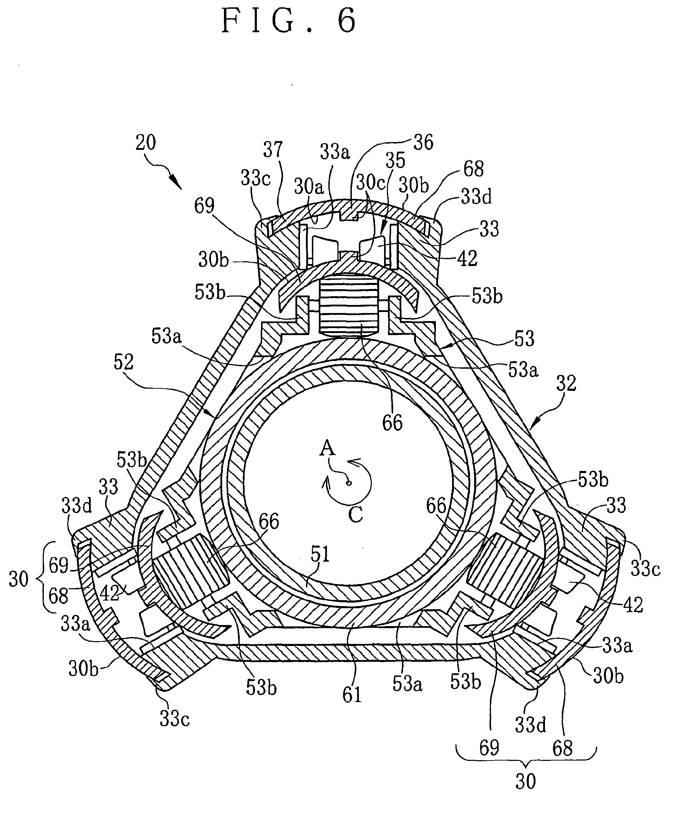

In Figs. 3, 4, 5 and 6, the housing sleeve 32 has a sleeve form of which a shape as viewed in a cross section is substantially a regular triangular prism with three flat side walls, with which three curved walls are combined alternately. The endless belts 30 are wound about the housing sleeve 32. A shaft sleeve 51 is disposed within the housing sleeve 32, and is mounted on the head assembly 11a of the endoscope 10 by receiving insertion.

The endless belts 30 are disposed on respectively curved walls of the housing sleeve 32. The endless belts 30 are supported on the housing sleeve 32 in an endlessly movable manner. To prepare each of the endless belts 30, a strip of a belt with ends is used at first, and positioned to wrap the housing sleeve 32. Then the ends of the belt are attached to one another by adhesive agent or thermal welding, to obtain the endless belts 30 supported on the housing sleeve 32.

Centrally raised support walls 33 of a strip form are formed with the housing sleeve 32, and support an upper belt run 68 of the endless belts 30. The support walls 33 have a height increasing from the edge areas to the center area with respect to the transverse direction of the upper belt run 68. Thus, the upper belt run 68 is supported in such an inverted U-shaped curve that its distance from the center of the shaft sleeve 51 decreases from a center portion 36 to lateral side portions 37 of the upper belt run 68. Tensioning devices 34 with an arcuate surface are secured to respectively proximal and distal ends of the support walls 33, and contact an inner surface 30a of bent portions of the endless belts 30 for the turn around. The tensioning devices 34 have a semicircular shape. A material for forming the tensioning devices 34 has sufficient smoothness for the endless belts 30 to turn around stably, for example, nylon. Various materials with low friction can be used for the tensioning devices 34, such as PEEK (polyetheretherketone), Teflon (trade name of polytetrafluoroethylene) and the like.

A holder opening 33a is formed in each of the support walls 33. A roller device 35 is fitted in the holder opening 33a, and contacts the endless belts 30 in an endlessly movable manner. The roller device 35 includes first, second and third belt rollers 41, 42 and 43 or pressure rollers, and a pair of support plates 40. The belt rollers 41-43 are arranged in the axial direction A. The support plates 40 keep the belt rollers 41-43 rotatable. Note that the belt rollers 41-43 may be directly supported on the housing sleeve 32.

The inner surface 30a of the endless belts 30 contacts the belt rollers 41-43. The center portion 36 of the endless belts 30 for contacting the belt rollers 41-43 has a larger thickness than the remaining portion of the endless belts 30, and has a higher rigidity than the remaining portion.

Roller grooves 41a, 42a and 43a are formed in respectively the belt rollers 41-43. A belt ridge 30c is formed to project from the inner surface 30a of the endless belts 30, and extends in the whole length of the endless belts 30. The belt ridge 30c is engaged with the roller grooves 41a, 42a and 43a in a slidable manner, and prevents the endless belts 30 from offsetting in the rotational direction C. Also, a groove 33b is formed in the support walls 33 and engaged with the belt ridge 30c in a slidable manner. A groove 34a is formed in the tensioning devices 34 and engaged with the belt ridge 30c in a turnable manner. Note that a coating of lubricant agent is applied to surfaces of the grooves 33b and 34a, the roller grooves 41a-43a and the belt ridge 30c for high smoothness in the contact.

A first side projection 33c and a second side projection 33d or side rail portions of a hook shape are formed on the support walls 33, and partially cover the lateral side portions 37 of the endless belts 30. A distance of the side projections 33c and 33d from the center of the shaft sleeve 51 is smaller than a distance of the center portion 36 of the endless belts 30 from the center of the shaft sleeve 51. The height of the side projections 33c and 33d is smaller than that of the center portion 36.

Also, the housing sleeve 32 contains a threaded sleeve 52 as a rotatable drive sleeve for transmission, and a support frame 53 or support sleeve. The threaded sleeve 52 is supported around the shaft sleeve 51 in a rotatable manner. The support frame 53 is disposed around the shaft sleeve 51 and the threaded sleeve 52.

A rear end ring 56 of a hexagonal form is secured to a proximal end of the support frame 53. A front guide ring 57 is fitted on a distal end of the support frame 53 for preventing incidental entry of tissue of a cavity wall of the gastrointestinal tract. A rear guide ring 58 is fitted on the rear end ring 56.

The threaded sleeve 52 supported around the shaft sleeve 51 rotates about an axis extending in the axial direction A. The threaded sleeve 52 has worm gear teeth 61 and spur gear teeth 62. The worm gear teeth 61 are a screw thread of a helical form. The spur gear teeth 62 are disposed at a proximal end of the threaded sleeve 52. A pinion 64 is connected with the coil structure 63, and meshed with the spur gear teeth 62. The pinion 64 is rotated by the coil structure 63, so that the threaded sleeve 52 is rotated by rotation of the spur gear teeth 62.

The support frame 53 as viewed in a cross section is in a hexagonal shape which is defined by removing triangular corner portions smaller than those of the housing sleeve 32. Also, the support frame 53 is positioned in a coaxial manner with the housing sleeve 32. Cutouts 53a are formed in six side walls of the support frame 53. Three of the cutouts 53a are opposed to the support walls 33 of the housing sleeve 32. Engagement rollers 66 or drive gears are positioned in the three of the cutouts 53a, and drive the endless belts 30. Among the engagement rollers 66, two are disposed in each one of the cutouts 53a. Pivot brackets 53b or pivot supports are formed on the support frame 53, and support the engagement rollers 66 in a rotatable manner. The engagement rollers 66 are disposed between the first and second belt rollers 41 and 42 and between the second and third belt rollers 42 and 43.

The engagement rollers 66 are meshed with the worm gear teeth 61 of the threaded sleeve 52, and contact the outer surface 30b of the endless belts 30. The engagement rollers 66 have such a form that their tooth surface is curved convexly in a U-shape by following the transverse curve of a lower belt run 69, and nip the lower belt run 69 together with the belt rollers 41-43 or pressure rollers. The engagement rollers 66 are overlapped with the belt rollers 41-43 in the radial direction of the housing sleeve 32. The lower belt run 69 is curved in a W shape between the belt rollers 41-43 and the engagement rollers 66.

A distal opening 53c is formed in the support frame 53, and receives entry of a distal end of the shaft sleeve 51.

The front guide ring 57 includes an annular ridge 57a and a guide flange 57b. The annular ridge 57a is fitted in the distal opening 53c. The guide flange 57b prevents a cavity wall of the gastrointestinal tract from entangling in the inside of the guide assembly 20. The guide flange 57b has a cup shape with a diameter increasing according to a distance from the annular ridge 57a. The shape of the guide flange 57b as viewed in a cross section is triangular with three additional curved walls in a manner similar to the housing sleeve 32, but slightly smaller than the housing sleeve 32.

The rear end ring 56 is formed in a hexagonal form similar to the support frame 53. A central opening 56a is defined in the rear end ring 56. A central lumen 51a is defined in the shaft sleeve 51, and communicates with the central opening 56a. A cutout 56b is formed in the rear end ring 56, and contains the pinion 64 in a rotatable manner. The pinion 64 is meshed with the spur gear teeth 62 of the threaded sleeve 52. A hole (not shown) is formed in the rear end ring 56, and receives insertion of the coil structure 63, which is connected to the pinion 64.

The rear guide ring 58 includes an annular ridge 58a and a guide flange 58b in a manner similar to the front guide ring 57. The annular ridge 58a is fitted in the central opening 56a of the rear end ring 56.

The operation of the guide assembly 20 is described now. At first, the head assembly 11a of the elongated tube 11 of the endoscope 10 is entered in the central lumen 51a of the shaft sleeve 51 to mount the guide assembly 20 on the head assembly 11a. Power sources of the processing apparatus, the light source apparatus and the input panel 26 are turned on. The imaging for the diagnosis is ready. Then the head assembly 11a of the endoscope 10 is entered in a gastrointestinal tract of a body of a patient.

The head assembly 11a is advanced to a predetermined site in the body cavity, for example, short of the sigmoid colon. Then the control button 27 of the input panel 26 is depressed to input a command signal for the propulsion. The motor 21 is driven to rotate the coil structure 63 in a predetermined direction. This causes the pinion 64 to rotate. The spur gear teeth 62 rotate to cause the threaded sleeve 52 to rotate.

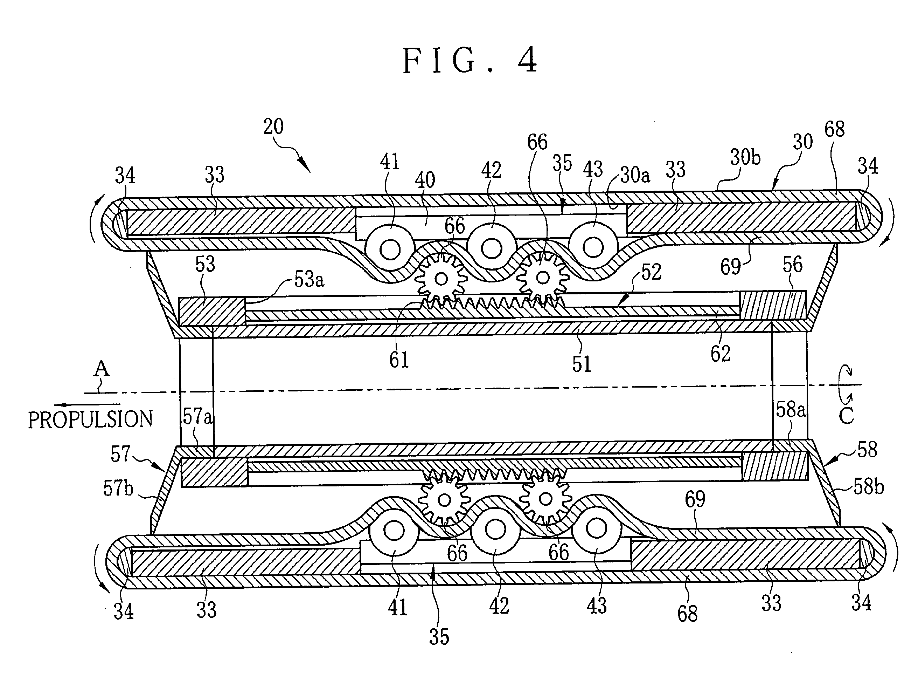

The rotation of the threaded sleeve 52 rotates the engagement rollers 66 or drive gears meshed with the worm gear teeth 61. Thus, the endless belts 30 nipped between the engagement rollers 66 and the belt rollers 41-43 are turned around in the direction of the arrow in Fig. 4. The outer surface 30b of the upper belt run 68 of the endless belts 30 outside the housing sleeve 32 is moved in the proximal direction in contact with the cavity wall of the body cavity. The outer surface 30b of the lower belt run 69 of the endless belts 30 inside the housing sleeve 32 is moved in the distal direction, so that the endless belts 30 are circulated.

The endless belts 30 contact the cavity wall of the gastrointestinal tract, and are circulated to exert force to move in the proximal direction of the head assembly 11a reverse to the distal direction. The guide assembly 20 applies the force to the body cavity from the distal side toward the proximal side, and propels the head assembly 11a of the endoscope 10 to move in the distal direction in the body cavity. If a doctor or operator wishes to move the guide assembly 20 in the proximal direction, he or she causes the endless belts 30 to operate in the directions reverse to those for the movement in the distal direction.

The lateral side portions 37 of the endless belts 30 are guided by the side projections 33c and 33d. When the guide assembly 20 propels the head assembly 11a in the body cavity, the lateral side portions 37 can be prevented from contacting a cavity wall of the body cavity. Only the center portion 36 of the endless belts 30 can contact the cavity wall. This is effective in preventing the cavity wall from entanglement on the inner surface 30a of the endless belts 30. The height of the side projections 33c and 33d is smaller than the height of the center portion 36 of the endless belts 30. So the endless belts 30 can run for the propulsion safely even if the side projections 33c and 33d should contact the cavity wall, because the force for propulsion at the center portion 36 is sufficiently strong.

When the speed button 28 of the input panel 26 is operated to input a command signal for change of the speed, rotational speed of the coil structure 63 is changed by controlling the motor 21. Thus, the moving speed of the guide assembly 20 is changed. When the control button 27 of the input panel 26 is operated to input a command signal for return, the coil structure 63 is rotated in a backward direction by controlling the motor 21. Thus, the guide assembly 20 is moved in the proximal direction. When the control button 27 is operated to input a command signal for stop, the coil structure 63 is stopped by stopping the motor 21. Thus, the guide assembly 20 is stopped. It is thus possible to advance the head assembly 11a of the endoscope 10 to a predetermined site in the body cavity as desired by a doctor or operator.

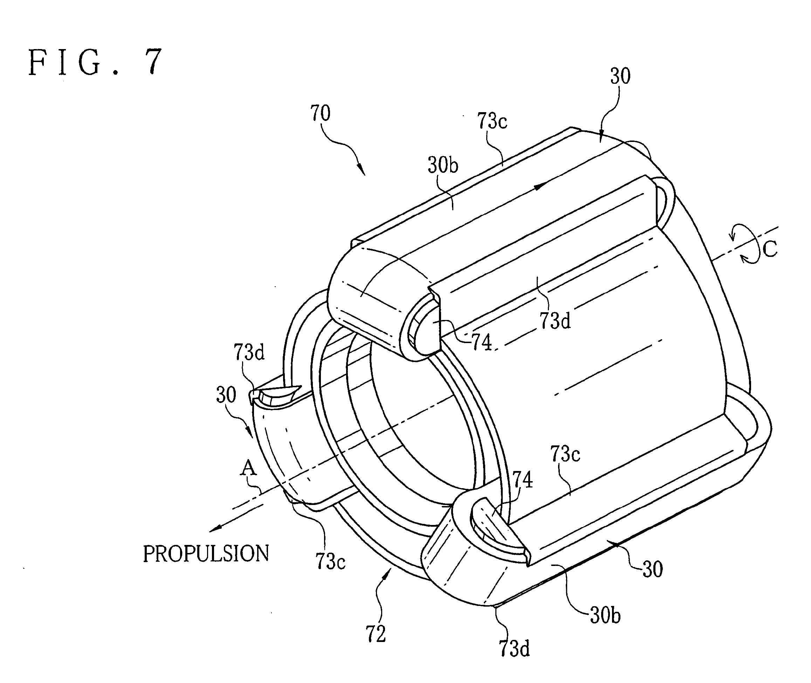

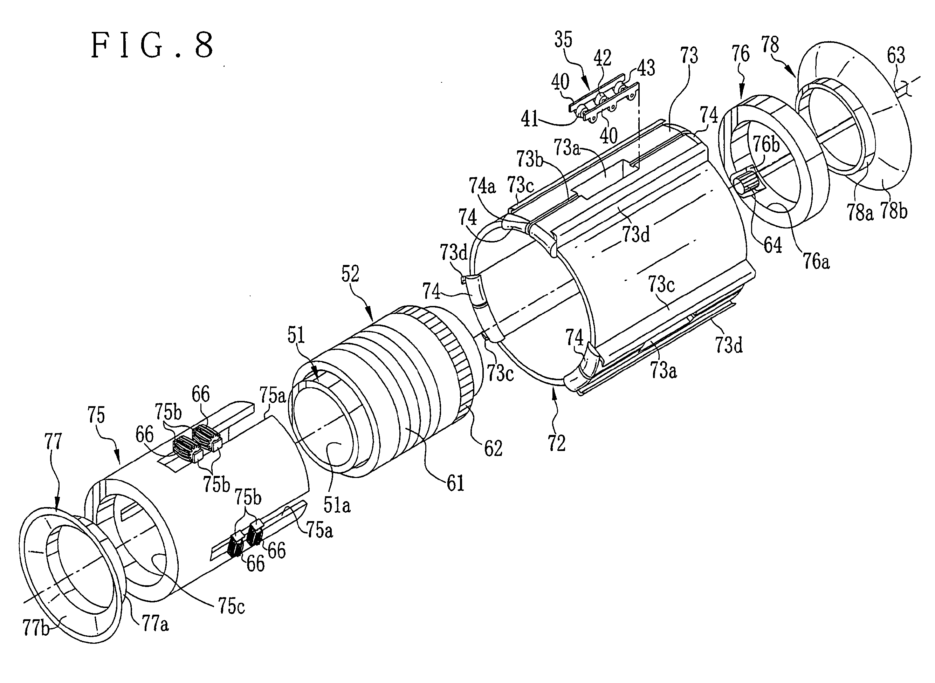

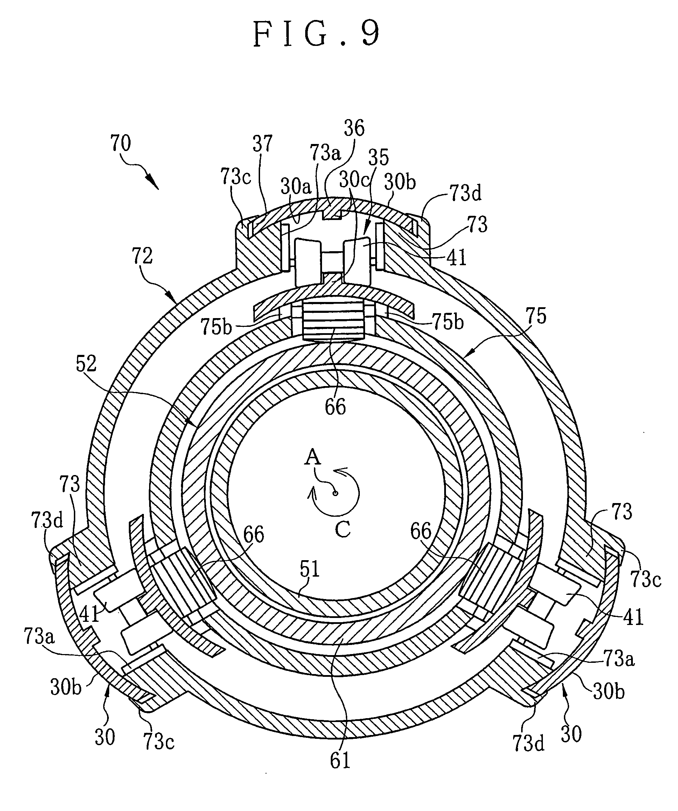

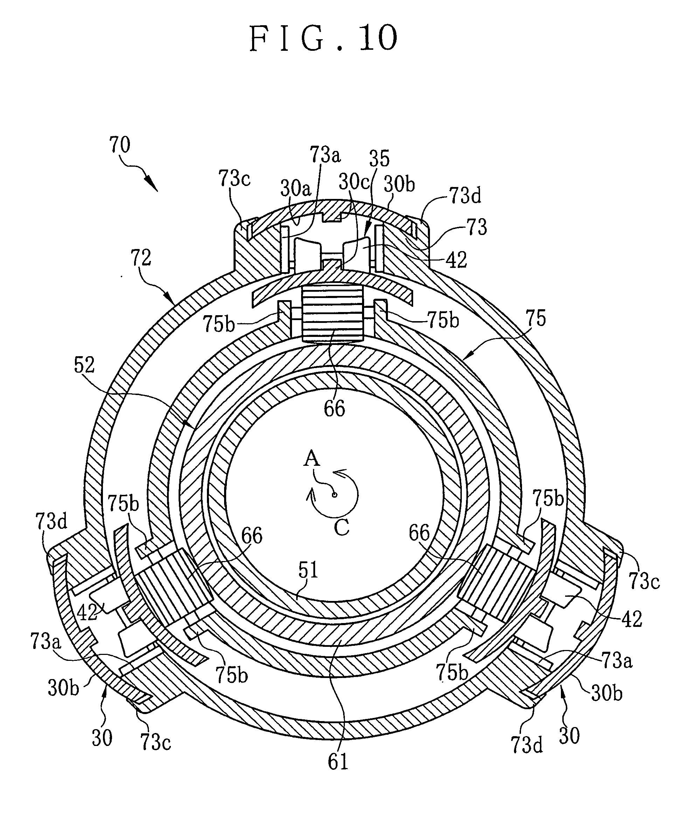

In Figs. 7-10, a guide assembly 70 of another preferred embodiment is illustrated, and includes a housing sleeve 72 of a cylindrical shape. Elements similar to those of the above embodiment are designated with identical reference numerals.

Centrally raised support walls 73 of a strip form are formed on the housing sleeve 72, and support respectively the endless belts 30. The support walls 73 have a height increasing from the edge areas to the center area with respect to the transverse direction of the endless belts 30. Thus, the endless belts 30 are supported in such an inverted U-shaped curve that their distance from the center of the shaft sleeve 51 decreases from the center portion 36 to the lateral side portions 37. Tensioning devices 74 with an arcuate surface are secured to respectively proximal and distal ends of the support walls 73, have a semicircular shape, and contact the inner surface 30a of bent portions of the endless belts 30.

A holder opening 73a is formed in each of the support walls 73. The roller device 35 is fitted in the holder opening 73a. A groove 73b is formed in each of the support walls 73. The belt ridge 30c of the endless belts 30 is engaged with and received in the groove 73b in a slidable manner. Similarly, a groove 74a is formed in each of the tensioning devices 74 for receiving the belt ridge 30c movably.

A first side projection 73c and a second side projection 73d or side rail portions are formed on the support walls 73, and guide the lateral side portions 37 of the endless belts 30. The height of the side projections 73c and 73d is smaller than that of the center portion 36 of the endless belts 30.

A support sleeve 75 or support frame is disposed in the housing sleeve 72, and contains the shaft sleeve 51 and the threaded sleeve 52. The support sleeve 75 is positioned coaxially with the housing sleeve 72.

A rear end ring 76 is secured to a proximal end of the support sleeve 75. A front guide ring 77 is secured to a distal end of the support sleeve 75 for preventing a cavity wall of a body cavity from entanglement in the guide assembly 20. A rear guide ring 78 is secured to the rear end ring 76.

Three cutouts 75a are formed in the support sleeve 75 and opposed to the support walls 73 of the housing sleeve 72. Two of the engagement rollers 66 are disposed in each one of the cutouts 75a. Pivot brackets 75b or pivot supports are formed on the support sleeve 75, and support the engagement rollers 66 in a rotatable manner.

A distal opening 75c is formed at a distal end of the support sleeve 75, and receives a distal end of the shaft sleeve 51.

The rear end ring 76 has a central opening 76a and a cutout 76b. The central opening 76a communicates with the central lumen 51a of the shaft sleeve 51. The cutout 76b contains the pinion 64 in a rotatable manner.

The front guide ring 77 includes an annular ridge 77a and a guide flange 77b. The annular ridge 77a is fitted in the distal opening 75c of the support sleeve 75. The guide flange 77b has a cup shape. The rear guide ring 78 includes an annular ridge 78a and a guide flange 78b. The annular ridge 78a is fitted in the central opening 76a of the rear end ring 76. The guide flange 78b has a cup shape.

The lateral side portions 37 of the endless belts 30 are guided by the side projections 73c and 73d, and thus can be prevented from contacting a cavity wall of the body cavity. Only the center portion 36 of the endless belts 30 can contact the cavity wall. This is effective in preventing the cavity wall from entanglement into the area of the inner surface 30a. The height of the side projections 73c and 73d is smaller than the height of the center portion 36 of the endless belts 30. So the endless belts 30 can run for the propulsion even if the side projections 73c and 73d should contact the cavity wall, because the force for propulsion at the center portion 36 is sufficiently strong.

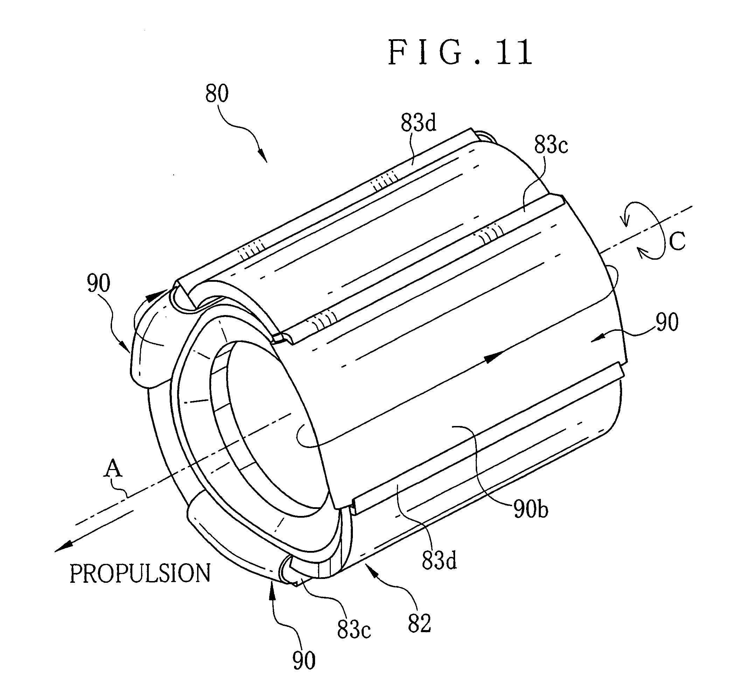

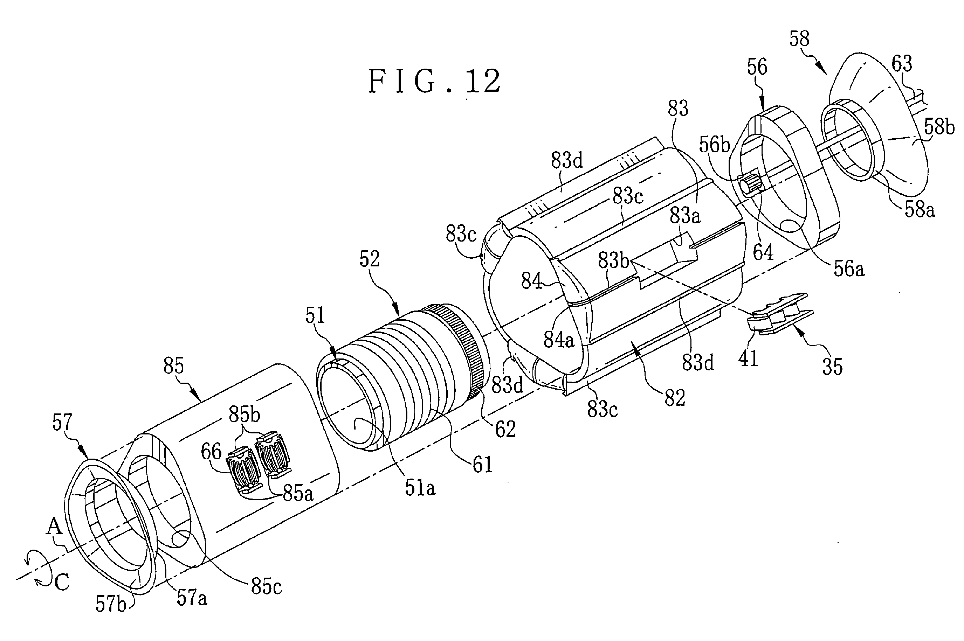

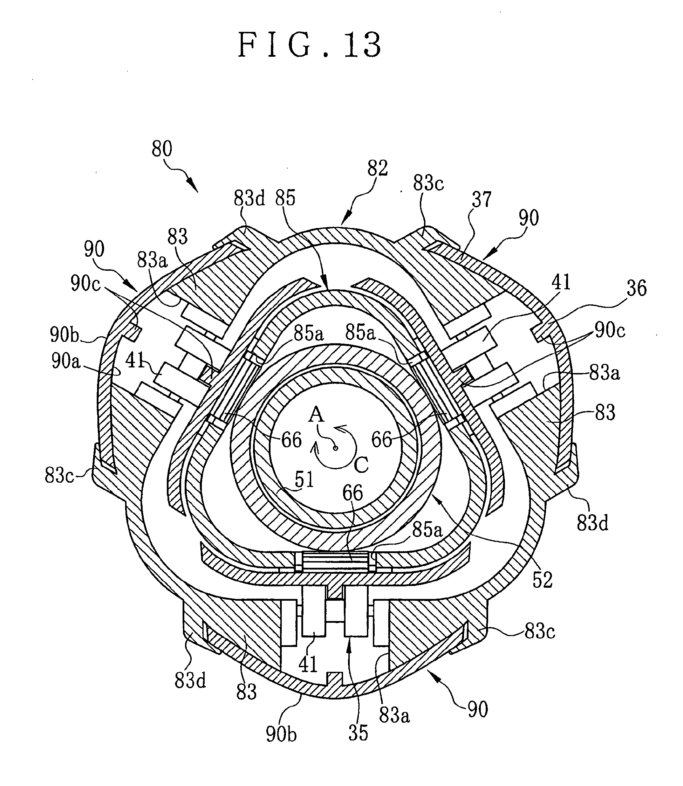

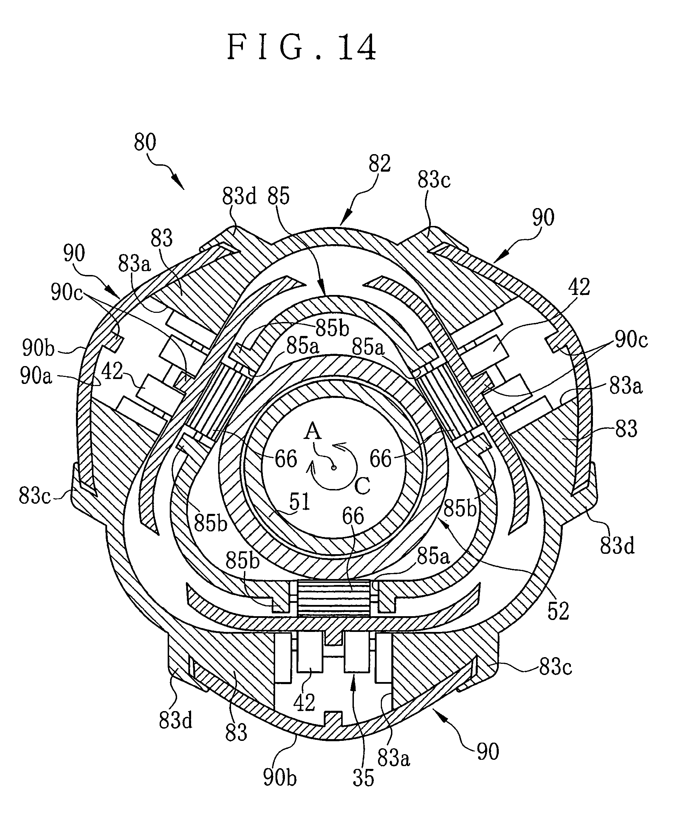

In Figs. 11-14, still another preferred guide assembly 80 is illustrated. Endless belts 90 are disposed on side walls of a housing sleeve 82. The housing sleeve 82 has three support walls with a large width, and three curved walls with a small width. The endless belts 90 have a considerably large width in comparison with the above embodiments. Elements similar to those of the above embodiments are designated with identical reference numerals.

Centrally raised support walls 83 of a strip form are formed on the housing sleeve 82, and support respectively the endless belts 90. The support walls 83 have a height increasing from the edge areas to the center area with respect to the transverse direction of the endless belts 90. Thus, the endless belts 90 are supported in such an inverted U-shaped curve that their distance from the center of the shaft sleeve 51 decreases from the center portion 36 to the lateral side portions 37. Tensioning devices 84 with an arcuate surface are secured to respectively proximal and distal ends of the support walls 83, have a semicircular shape, and contact an inner surface 90a of bent portions of the endless belts 90.

A holder opening 83a is formed in each of the support walls 83. The roller device 35 is fitted in the holder opening 83a. A groove 83b is formed in each of the support walls 83. A belt ridge 90c is formed to project from each of the endless belts 90, and engaged with the groove 83b in a slidable manner. A groove 84a is formed in each of the tensioning devices 84, and engaged with the belt ridge 90c in a turnable manner. Also, the endless belts 90 have an outer surface 90b.

A first side projection 83c and a second side projection 83d or side rail portions are formed with the support walls 83, and guide the lateral side portions 37 of the endless belts 90 by covering partially. A height of the side projections 83c and 83d is smaller than a height of the center portion 36 of the endless belts 90. In short, the endless belts 90 are bent longitudinally so that the center portion 36 projects over the level of the side projections 83c and 83d.

A support sleeve 85 or support frame of a shape of a triangular prism is disposed in the housing sleeve 82, and contains the shaft sleeve 51 and the threaded sleeve 52. The support sleeve 85 is positioned coaxially with the housing sleeve 82.

Support openings 85a are formed in respectively flat walls of the support sleeve 85. The engagement rollers 66 are contained in the support openings 85a. Pivot brackets 85b or pivot supports are formed on the support sleeve 85, and support the engagement rollers 66 in a rotatable manner in the support openings 85a.

A distal opening 85c is formed in the support sleeve 85, and receives insertion of a distal end of the shaft sleeve 51. Also, the annular ridge 57a of the front guide ring 57 is inserted in the distal opening 85c. The rear end ring 56 is secured to a proximal end of the support sleeve 85. The rear guide ring 58 is secured to the rear end ring 56.

The lateral side portions 37 of the endless belts 90 are guided by the side projections 83c and 83d, and thus can be prevented from contacting a cavity wall of the body cavity. Only the center portion 36 of the endless belts 90 can contact the cavity wall. This is effective in preventing the cavity wall from entanglement into the area of the inner surface 90a. The height of the side projections 83c and 83d is smaller than the height of the center portion 36 of the endless belts 90. So the endless belts 90 can run for the propulsion even if the side projections 83c and 83d should contact the cavity wall, because the force for propulsion at the center portion 36 is sufficiently strong.

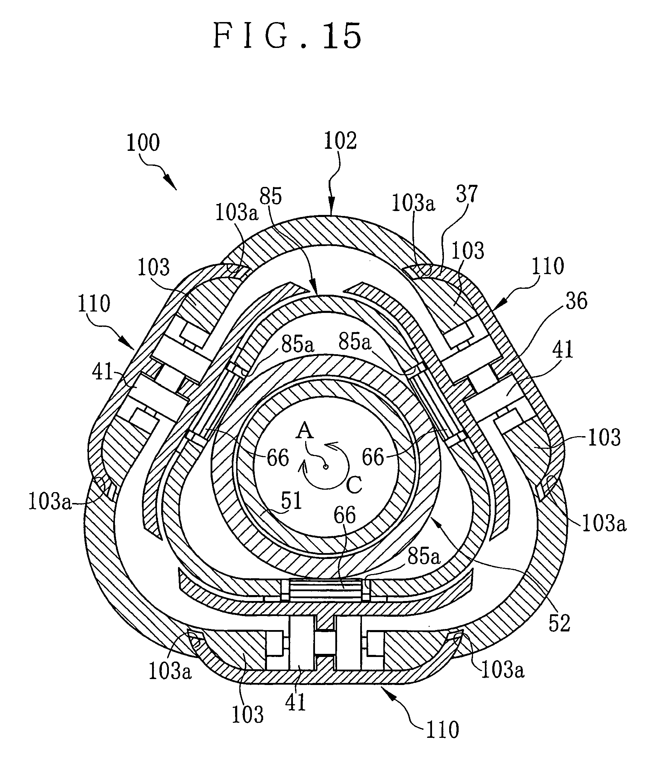

In Fig. 15, another preferred embodiment of a housing sleeve 102 is illustrated. A side rail channel 103a or groove as side rail portion is formed along each of edges of centrally raised support walls 103 of a strip form. Endless belts 110 have the lateral side portions 37, each of which is inserted in the side rail channel 103a. This is effective in preventing lateral side portions 37 from contacting a cavity wall of the gastrointestinal tract.

In the above embodiments, the side projections guide the lateral side portions 37 of the endless belts supported by the support walls. Furthermore, side projections may be formed partially to cover the lateral side portions 37 of the endless belts positioned at the tensioning devices.

In the above embodiments, the first and second side projections are formed originally on the housing sleeve. However, the first and second side projections can be previously formed with a cover separately prepared, before the cover can be retained on the housing sleeve.

In the above embodiments, the threaded sleeve and drive sleeve are formed in the triangular, hexagonal and circular shapes as viewed in a cross section. However, those can be formed in other shapes, such as quadrangular, pentagonal and otherwise polygonal shapes.

In the above embodiments, the shape of the upper belt run 68 as viewed in a cross section is an inverted U-shape of which the center portion 36 is curved with a higher distance from the center axis than the lateral side portions 37. However, the shape of the upper belt run 68 of the endless belts as viewed in a cross section can be a flat shape of a rectangular quadrilateral.

In the above embodiments, the support walls of the strip form have a height increasing from the edge area to the center area with respect to the transverse direction of the endless belts. The height of the side projections is smaller than that of the center portion 36 of the endless belts. However, the side projections can have a larger height than that of the center portion 36 of the endless belts.

In the above embodiments, the support walls 33, 73, 83 and 103 have the center area with a larger height than their edge areas. The tensioning devices have the center area with a height equal to that of their edge areas. Alternatively, a height of the support walls may be decreased gradually toward the tensioning devices of the proximal and distal ends, so that a difference between the center area and the edge areas in the height can be decreased in the axial direction. Also, the tensioning devices may have a center area with a larger height than their edge areas in compliance with the support walls of the embodiments. Also, support walls may have a center area with a height equal to that of their edge areas. In other words, the support walls can be formed flatly or with a cylindrical surface very near to a flat surface.

In the above embodiments, the engagement rollers 66 are rotated by use of the worm gear teeth 61 in the drive sleeve for transmission, so as to drive the endless belts to turn around. However, it is possible for the worm gear teeth 61 or threaded sleeve to drive the endless belts directly without the engagement rollers 66. Note that a rotational direction of the worm gear teeth 61 or threaded sleeve for the propulsion without using the engagement rollers 66 is reverse to that of the worm gear teeth 61 for the propulsion in combination with the engagement rollers 66. A relationship between a rotational direction of the coil structure and the proximal and distal directions for moving the endoscope according to the button panel must be changed suitably for the purpose of the propulsion.

Also, a pair of rotatable support rollers can be used instead of the tensioning devices 34 for keeping the endless belts movable endlessly.

In the above embodiments, the endoscope is for a medical use. However, an endoscope of the invention can be one for industrial use, a probe of an endoscope, or the like for various purposes.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A guide assembly for an endoscope (10) including a section of an elongated tube for entry in a body cavity, comprising:
a shaft sleeve (51) for mounting on said elongated tube;
a housing sleeve (32, 72, 82, 102) supported around said shaft sleeve;
plural endless belts (30, 90, 110), secured to said housing sleeve, for endlessly moving in an axial direction (A) of said elongated tube, and propelling said elongated tube by contacting a cavity wall of said body cavity;
a driving device (61, 66), contained in said housing sleeve, for driving said endless belts;
**characterised in** further comprising
first and second side rail portions (33c, 33d, 73c, 73d, 83c, 83d, 103a), disposed on said housing sleeve, for covering lateral side portions (37) of each of said endless belts at least partially, and guiding said endless belts.

2. A guide assembly as defined in claim 1, wherein said first and second side rail portions cause curving of a center portion of said endless belts outwards in a raised form with respect to a transverse direction thereof.

3. A guide assembly as defined in claim 1, further comprising a centrally raised support wall, formed together with said first and second side rail portions, for raising a center portion of said endless belts with respect to a transverse direction thereof outwards further than said lateral side portions.

4. A guide assembly as defined in claim 1, wherein said side rail portions are formed together with said housing sleeve.

5. A guide assembly as defined in claim 1, wherein said side rail portions are secured to said housing sleeve.

6. A guide assembly as defined in claim 4, wherein each of said first and second side rail portions includes a side rail channel for receiving entry of one of said lateral side portions.

7. A guide assembly as defined in claim 6, wherein said side rail channel is inclined to cause curving of a center portion of said endless belts outwards in a raised form with respect to a transverse direction thereof.

8. A guide assembly as defined in claim 1, wherein each of said endless belts includes:
an upper belt run, disposed outside said housing sleeve, having an outer surface, for contacting said cavity wall;
a lower belt run disposed inside said housing sleeve.

9. A guide assembly as defined in claim 2, wherein said driving device includes:
a drive sleeve, contained in said housing sleeve, and supported around said shaft sleeve in a rotatable manner;
worm gear teeth formed around said drive sleeve;
an engagement roller, having gear teeth, meshed with said worm gear teeth, for turning around said endless belts by engagement therewith.

10. A guide assembly as defined in claim 9, further comprising plural belt rollers, secured to said housing sleeve in a rotatable manner, for pressing each of said endless belts for contact with said engagement roller.

11. A guide assembly as defined in claim 10, wherein a portion of said endless belts in contact with said belt rollers has higher rigidity than a remaining portion thereof.

12. A guide assembly as defined in claim 9, wherein a roller surface of said engagement roller is curved according to a curve of said endless belts.

13. A guide assembly as defined in claim 10, wherein a roller surface of said belt rollers is curved according to a curve of said endless belts.

## Patentansprüche

1. Führungsanordnung für ein Endoskop (10) mit einem Abschnitt eines länglichen Schlauchs zum Einführen in einen Körperhohlraum, umfassend:
eine Schafthülse (51) zur Lagerung an dem länglichen Schlauch;
eine Gehäusehülse (32, 72, 82, 102), die um die Schafthülse herum gelagert ist;
mehrere Endlosriemen (30, 90, 110), die an der Gehäusehülse befestigt sind, um sich endlos in einer axialen Richtung (A) des länglichen Schlauchs zu bewegen und den länglichen Schlauch unter Berührung einer Hohlraumwand des Körperhohlraums vorzutreiben;
eine Antriebseinrichtung (61, 66), die in der Gehäusehülse enthalten ist, um die Endlosriemen anzutreiben;
weiterhin **gekennzeichnet durch**:
erste und zweite seitliche Schienenabschnitte (33c, 33d, 73c, 73d, 83c, 83d, 103a), die an der Gehäusehülse angeordnet sind, um laterale Seitenabschnitte (37) jedes der Endlosriemen zumindest teilweise abzudecken und die Endlosriemen zu führen.

2. Führungsanordnung nach Anspruch 1, bei der die ersten und zweiten seitlichen Schienenabschnitte eine Krümmung eines Mittelabschnitts der Endlosriemen nach außen in einer angehobenen Form bezüglich ihrer Querrichtung veranlassen.

3. Führungsanordnung nach Anspruch 1, weiterhin umfassend eine mittig angehobene Trägerwand, die zusammen mit den ersten und zweiten seitlichen Schienenabschnitten ausgebildet ist, um einen Mittelbereich der Endlosriemen bezüglich ihrer Querrichtung weiter nach außen anzuheben als die lateralen Seitenabschnitte.

4. Führungsanordnung nach Anspruch 1, bei der die seitlichen Schienenabschnitte zusammen mit der Gehäusehülse ausgebildet sind.

5. Führungsanordnung nach Anspruch 1, bei der die seitlichen Schienenabschnitte an der Gehäusehülse festgelegt sind.

6. Führungsanordnung nach Anspruch 4, bei der jeder von den ersten und zweiten seitlichen Schienenabschnitten einen seitlichen Schienenkanal aufweist zum Aufnehmen des Eintritts eines der lateralen Seitenabschnitte.

7. Führungsanordnung nach Anspruch 6, bei der der Schienenkanal geneigt ist, um eine Krümmung eines Mittelbereichs der Endlosriemen nach außen in angehobener Form bezüglich ihrer Querrichtung zu veranlassen.

8. Führungsanordnung nach Anspruch 1, bei der jeder der Endlosriemen enthält:
einen oberen Riementrum, angeordnet außerhalb der Gehäusehülse mit einer Außenfläche zur Berührung mit der Hohlraumwand;
einen unteren Riementrum, der im Inneren der Gehäusehülse befindlich ist.

9. Führungsanordnung nach Anspruch 2, bei der die Antriebseinrichtung enthält:
eine Antriebshülse, die in der Gehäusehülse enthalten ist und um die Schafthülse drehbar gelagert ist;
ein Schneckenrad, das um die Antriebshülse herum ausgebildet ist;
eine verzahnte Eingriffsrolle, die mit dem Schneckenrad kämmt, um die Endlosriemen durch Eingriff mit ihnen umzuwälzen.

10. Führungsanordnung nach Anspruch 9, weiterhin umfassend mehrere Riemenrollen, die an der Gehäusehülse drehbar angebracht sind, um jeden der Endlosriemen zur Berührung mit der Eingriffsrolle anzudrücken.

11. Führungsanordnung nach Anspruch 10, bei der ein Abschnitt der Endlosriemen, der mit den Riemenrollen in Berührung steht, eine höhere Steifigkeit aufweist als deren übriger Teil.

12. Führungsanordnung nach Anspruch 9, bei der eine Rollenoberfläche der Eintrittsrolle entsprechend einer Krümmung der Endlosriemen gekrümmt ist.

13. Führungsanordnung nach Anspruch 10, bei der eine Rollenoberfläche der Riemenrollen gemäß einer Krümmung der Endlosriemen gekrümmt ist.

## Revendications

1. Ensemble de guidage pour un endoscope (10) comprenant une section d'un tube allongé pour l'entrée dans une cavité corporelle, comportant :
un manchon d'arbre (51) pour montage sur ledit tube allongé ;
un manchon de logement (32, 72, 82, 102) supporté autour dudit manchon d'arbre ;
plusieurs bandes sans fin (30, 90, 110), fixées sur ledit manchon de logement, afin de se déplacer sans fin dans une direction axiale (A) dudit tube allongé, et propulser ledit tube allongé en entrant en contact avec une paroi de cavité de ladite cavité corporelle ;
un dispositif d'entraînement (61, 66), contenu dans ledit manchon de logement, afin d'entraîner lesdites bandes sans fin ;
**caractérisé en ce qu'**il comporte en outre
des première et deuxième parties de rail latéral (33c, 33d, 73c, 73d, 83c, 83d, 103a), disposées sur ledit manchon de logement, afin de recouvrir au moins partiellement des parties latérales (37) de chacune desdites bandes sans fin, et de guider lesdites bandes sans fin.

2. Ensemble de guidage selon la revendication 1, dans lequel lesdites première et deuxième parties de rail latéral provoquent une courbure vers l'extérieur d'une partie centrale desdites bandes sans fin sous une forme relevée par rapport à une direction transversale de celles-ci.

3. Ensemble de guidage selon la revendication 1, comportant en outre une paroi de support relevée de manière centrale, formée avec lesdites première et deuxième parties de rail latéral, afin de relever une partie centrale desdites bandes sans fin par rapport à une direction transversale de celles-ci davantage vers l'extérieur que lesdites parties latérales.

4. Ensemble de guidage selon la revendication 1, dans lequel lesdites parties de rail latéral sont formées avec ledit manchon de logement.

5. Ensemble de guidage selon la revendication 1, dans lequel lesdites parties de rail latéral sont fixées sur ledit manchon de logement.

6. Ensemble de guidage selon la revendication 4, dans lequel chacune desdites première et deuxième parties de rail latéral comprend un canal de rail latéral destiné à recevoir l'entrée d'une desdites parties latérales.

7. Ensemble de guidage selon la revendication 6, dans lequel ledit canal de rail latéral est incliné afin de provoquer la courbure vers l'extérieur d'une partie centrale desdites bandes sans fin sous une forme relevée par rapport à une direction transversale de celles-ci.

8. Ensemble de guidage selon la revendication 1, dans lequel chacune desdites bandes sans fin comprend :
un brin supérieur de bande, disposé à l'extérieur dudit manchon de logement, ayant une surface extérieure, destiné à venir en contact ledit mur de cavité ;
un brin inférieur de bande disposé à l'intérieur dudit manchon de logement.

9. Ensemble de guidage selon la revendication 2, dans lequel ledit dispositif d'entraînement comprend :
un manchon d'entraînement, contenu dans ledit manchon de logement, et supporté autour dudit manchon d'arbre d'une manière rotative ;
des dents d'engrenage à vis sans fin formées autour dudit manchon d'entraînement ;
un rouleau d'engagement, ayant des dents d'engrenage, en prise avec lesdites dents d'engrenage à vis sans fin, destiné à tourner autour desdites bandes sans fin par engagement avec celles-ci.

10. Ensemble de guidage selon la revendication 9, comportant en outre plusieurs rouleaux de bande, fixés sur ledit manchon de logement de manière rotative, afin de pousser chacune desdites bandes sans fin pour venir en contact avec ledit rouleau d'engagement.

11. Ensemble de guidage selon la revendication 10, dans lequel une partie desdites bandes sans fin en contact avec lesdits rouleaux de bande a une rigidité plus élevée qu'une partie restante de celles-ci.

12. Ensemble de guidage selon la revendication 9, dans lequel une surface de rouleau dudit rouleau d'engagement est courbée suivant une courbe desdites bandes sans fin.

13. Ensemble de guidage selon la revendication 10, dans lequel une surface de rouleau desdits rouleaux de bande est courbée suivant une courbe desdites bandes sans fin.
